# EUROPEAN PATENT SPECIFICATION

(11) **EP 2 817 092 B1**
(45) Date of publication and mention of the grant of the patent: **30.11.2022**
(21) Application number: 13705476.3
(22) Date of filing: 22.02.2013
(51) Int. Cl.: B01J 23/60, B01J 37/02, B01J 35/00, C07C 29/17

(54) **METAL POWDERDOUS CATALYST COMPRISING A FE-ALLOY**
PULVERFÖRMIGER METALLKATALYSATOR MIT FE-LEGIERUNG
CATALYSEUR EN POUDRE MÉTALLIQUE COMPORTANT UN ALLIAGE DU FER

(30) Priority: 24.02.2012 EP 12156836
(43) Date of publication of application: 31.12.2014
(73) Proprietor: Dsm Ip Assets B.V., 6411 The Heerlen (NL)
(72) Inventor: BONRATH, Werner, CH-4002 Basel (CH); BUSS, Axel, CH-4002 Basel (CH)
(74) Representative: Kurt, Manfred
(86) International application number: PCT/EP2013/053513
(87) International publication number: WO 2013/124393

(56) References cited:
- WO-A1-2011/092280
- WO-A1-2012/001166
- WO-A2-2008/101603
- TW-A- 201 202 443
- SEMAGINA ET AL: "Structured catalyst of Pd/ZnO on sintered metal fibers for 2-methyl-3-butyn-2-ol selective hydrogenation", JOURNAL OF CATALYSIS, ACADEMIC PRESS, DULUTH, MN, US, vol. 251, no. 1, 7 September 2007 (2007-09-07), pages 213-222, XP022235097, ISSN: 0021-9517, DOI: 10.1016/J.JCAT.2007.06.028
- TRIBOLET P ET AL: "Palladium on carbon nanofibers grown on metallic filters as novel structured catalyst", CATALYSIS TODAY, ELSEVIER, NL, vol. 105, no. 3-4, 15 August 2005 (2005-08-15), pages 337-343, XP004998972, ISSN: 0920-5861, DOI: 10.1016/J.CATTOD.2005.06.035

## Description

The present invention is related to a new metal powder catalytic system comprising a Fe-alloy as a carrier and its use in hydrogenation processes.

Powderous catalysts are well known and used in chemical reactions. Important types of such catalysts are i.e. the Lindlar catalysts.

A Lindlar catalyst is a heterogeneous catalyst which consists of palladium deposited on a calcium carbonate carrier which is also treated with various forms of lead.

Such catalysts are of such an importance that there is always a need for their improvement.

WO 2012/001166 A1 discloses a hydrogenation catalyst comprising sintered metal fibers as carrier.

The goal of the present invention was to find a powderous catalyst with improved properties.

The powderous catalytic system according to the present invention does have a metal alloy as carrier material, instead of a calcium carbonate carrier.

This metal alloy is coated by a metal oxide layer on which palladium (Pd) is deposited.

Furthermore the new powderous catalytic system according to the present invention is free from lead (Pb).

This new powderous catalytic system has numerous advantages:
∘ The catalyst is easy to recycle (and to remove) after the reaction. This can be done i.e. by filtration.
∘ The catalyst can be used more than once (re-usable).
∘ The catalyst as such is a very stable system. It is i.e. stable in regard to acids as well as to water.
∘ The catalyst is easy to produce.
∘ The catalyst is easy to handle.
∘ The hydrogenation can be carried out without any solvents.
∘ The catalyst is free from lead.
∘ The catalyst shows high selectivity in hydrogenation reactions.

The catalytic system is in the form of a powder.

The metal alloy used as a carrier is preferably stainless steel.

Three main types of stainless steels are known. These are classified by their crystalline structure:
austenitic, ferritic, and martensitic.

The most important type (more than 70 % of the stainless steel production volume) is the austenitic steel. Austenitic steels have austenite as their primary phase. These are alloys containing usually between 18 wt-% - 20 wt-%, based on the total weight of the alloy, of chromium and 8 wt-% - 10 wt-%, based on the total weight of the alloy, of nickel.

Ferritic steels have ferrite as their main phase. These steels contain iron and about 17 wt-%, based on the total weight of the alloy, of chromium.

Martensitic steel has a characteristic orthorhombic martensite microstructure. Martensitic steels are low carbon steels.

Stainless steel can comprise further metals, such as i.e. Cu, Mn, Si, Mo, Ti, Al and Nb.

Furthermore stainless steel can comprise carbon as well.

Therefore the present invention relates to a powderous catalytic system wherein the metal alloy is stainless steel comprising
(i) 60 wt-% - 80 wt-%, based on the total weight of the stainless steel carrier, of Fe, and
   (ii) 12 wt-% - 25 wt-%, based on the total weight of the stainless steel carrier, of Cr, and
   (iii) 1 wt-% - 8 wt-%, based on the total weight of the stainless steel, of Ni, and
   (iv) 1 wt-% - 8 wt-%, based on the total weight of the stainless steel carrier, of Cu, and
   wherein the stainless steel is coated by a metal oxide layer impregnated with Pd as defined in claim 1.

Stainless steel is commercially available from many producers and traders. It can be bought i.e. from companies such as Sverdrup Hanssen, Nichelcrom Acciai Inox S.p.A or EOS GmbH.

Suitable products are i.e. EOS StainlessSteel GP1^{®} from EOS GmbH (Germany) The metal oxide layer, which coats the metal alloy, is non-acidic (preferably basic or amphoteric). The oxide layer comprises ZnO and Al.

The metal alloy is preferably coated with a thin layer of ZnO (0.5 - 3.5 µm thickness) and Al oxide.

Preferred is also a powderous catalytic system, wherein the non-acidic metal oxide layer is essentially free from Pb.

The coating of the metal alloy is done by commonly known processes, such as i.e. dip-coating.

Usually powderous catalytic system of the present invention comprises between 0.1 wt-% and 50 wt-%, based on the total weight of the catalyst, of ZnO, preferably between 0.1 wt-% and 30 wt-%, more preferably between 1.5 wt-% and 10 wt-% and most preferably between 2 wt-% and 8 wt-%.

Therefore the present invention also relates to a powderous catalytic system wherein the powderous catalytic system comprises between 0.1 wt-% and 50 wt-%, based on the total weight of the powderous catalytic system of ZnO (preferably between 0.1 wt-% and 30 wt-%, more preferably between 1.5 wt-% and 10 wt-% and most preferably between 2 wt-% and 8 wt-%).

The present invention relates to a powderous catalytic system wherein the metal oxide is a mixture of ZnO and Al2O3 and wherein the ratio of ZnO : Al2O3 is from 2:1 to 1:2 (preferably 1:1).

The coated metal alloys are then impregnated by Pd-nanoparticles. The nanoparticles are synthesized by commonly known methods, i.e. by using PdCl₂ as a precursor, which is then reduced by hydrogen.

Usually the Pd-nanoparticles, which are on the non-acidic metal oxide layer, have an average particle size of between 0.5 and 20 nm, preferably of between 2 and 15 nm, more preferably of between 5 and 12 nm and most preferably of between 7 to 10 nm. (The size is measured by light scattering methods).

Therefore the present invention also relates to a powderous catalytic system, wherein the Pd-nanoparticles have an average particle size of between 0.5 and 20 nm (preferably of between 2 and 15 nm, more preferably of between 5 and 12 nm and most preferably of between 7 to 10 nm).

The powderous catalytic system according to present invention comprises between 0.001 wt-% and 5 wt-%, based on the total weight of the catalyst, of the Pd- nanoparticles, preferably between 0.01 wt-% and 2 wt-% more preferably between 0.05 wt-% and 1 wt-%.

Therefore the present invention also relates to a powderous catalytic system, wherein the powderous catalytic system comprises between 0.001 wt-% and 5 wt-%, based on the total weight of the catalyst, of the Pd- nanoparticles (preferably between 0.01 wt-% and 2 wt-% more preferably between 0.05 wt-% and 1 wt-%).

The powderous catalytic system is usually activated before the use. The activation is done by using well known processes, such thermo activation in H₂.

The powderous catalytic system of the present invention is used in selective catalytic hydrogenation of organic starting material, especially of organic starting material comprising a carbon-carbon triple bond, more especially of alkynol compounds.

Therefore the present invention also relates to the use of a powderous catalytic system in selective catalytic hydrogenation of organic starting material, especially of organic starting material comprising a carbon-carbon triple bond, more especially of alkynol compounds.

Preferably the present invention relates to a process of reacting a compound of formula (I) 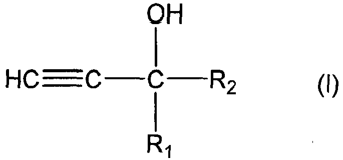 wherein
R₁ is linear or branched C₅-C₃₅ alkyl or linear or branched C₅-C₃₅ alkenyl moiety, wherein the C chain can be substituted, and
R₂ is linear or branched C₁-C₄ alkyl, wherein the C chain can be substituted, with hydrogen in the presence of the powderous catalytic system of the invention.

Hydrogen is usually used in the form H₂ gas.

Preferred compounds of formula (I) are the following: 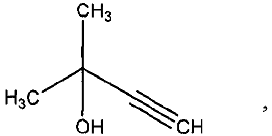 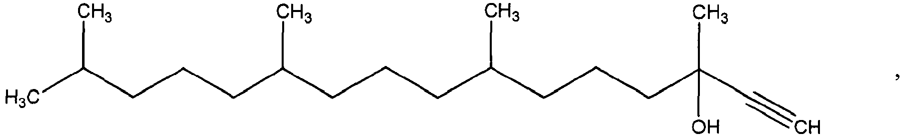 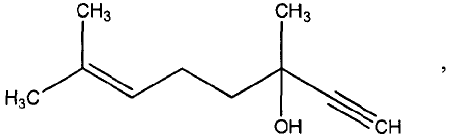 and

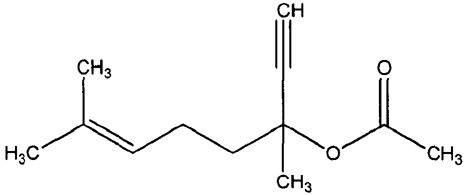

The following examples serve to illustrate the invention. All percentages are related to weight and the temperatures are given in degree Celsius, if not otherwise stated.

### Examples

### Example 1: Synthesis of the powderous catalytic system (stainless steel coated by Al₂O₃/ZnO and Pd deposition)

### Step 1: Thermal pre-treatment

The stainless steel powder (EOS StainlessSteel GP1^{®} commercially available from EOS GmbH, Germany) was subjected to a thermal pre-treatment at 450°C for 3 h.

### Step 2 Deposition of ZnO + Al₂O₃ (coating of the metal alloy carrier)

To a 100 ml-flask 20.0 g (53.3 mMol) of Al(NO₃)₃ 9H₂O and 70 ml of water were added. The mixture was stirred until the Al(NO₃)₃·9H₂O was completely dissolved. The solution was heated up to 95° C. Then 4.34 g (53.3 mMol) of ZnO powder was slowly added to the reaction solution. Heating and stirring were maintained until the ZnO was completely dissolved. The solution was then cooled down to room temperature and filtrated through a membrane filter.

The deposition of ZnO/Al₂O₃ was performed by adding the oxidized stainless steel powder (23.4 g) from step 1 to the precursor solution and stirring the mixture at room temperature for 15 min.

The powder was then filtered off via a membrane filter and dried in air at 40° C and 125 mbar for 2h followed by a calcination step at 450° C for 1h. The stirring-drying-calcination cycle was repeated 3 times. Finally, the powder support was calcined in air at 550° C for 1h.

22.75 g of coated stainless steel powder was obtained.

### Step 3: Preparation and deposition of the Pd-nanoparticles

318 mg (1.31 mmol) of sodium molybdate dihydrate and 212 mg (1.20 mmol) of palladium(II) chloride anhydrous were added to 60 ml of deionized water under heating (ca. 95°C). The mixture was stirred. The heating and stirring were continued until complete evaporation of the water (solid residue was formed). Afterwards, 60 ml of deionized water were added to the residue under stirring. The evaporation-dissolving cycle was repeated two times in order to completely dissolve PdCl2. Finally, 100 ml of hot water were added to the solid residue. The deep brown solution was cooled down to room temperature and filtrated through a paper filter. The filter was washed with water until the final volume of the precursor solution was 120 mL.

Afterwards the Pd° suspension was formed by bubbling hydrogen through the precursor solution for 1 h in a glass cylinder at room temperature.

The so obtained Pd° suspension and 22.75 g of the coated stainless steel powder (from step 2) were added to a 200 ml-flask. The mixture was stirred at room temperature for 15 min. The powder was filtered off via a filter paper and dried in air at 40° C and 125 mbar for 2 h. This process was repeated twice.

### Step 4: Thermo activation of the catalyst in H₂

The powderous catalytic system obtained from step 3 was subjected to a temperature treatment at 300° C for 4 h under H₂-Ar flow. Then, it was cooled down to room temperature under the same H₂-Ar flow. 20.3 g of the powderous catalyst according to the present invention was obtained.

### Example 2a: Selective hydrogenation of MBY to MBE

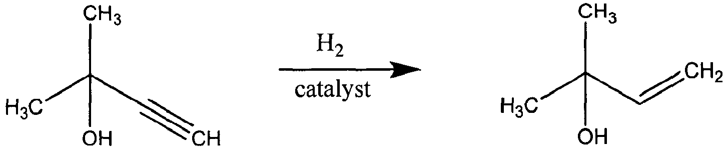

To 285 g (3.38 Mol) of MBY 1.5 g of the powderous catalytic system of Example 1 was added under stirring. The reaction was carried out at 45°C and 4 bar pressure.

The reaction was repeated four times under the same condition.

At the end of the reaction (after about 23 hours) the selectivity of the reaction was between 91.6 and 95.6 % and the conversion was between 99.4 and 99.9%.

It can be seen that the new powderous catalytic system has excellent properties as a catalyst for selective hydrogenations.

### Example 2b: Repeated selective hydrogenation of MBY to MBE

The same reaction conditions as in Example 2a have been used. At the end of the reaction (after about 13 - 19 hours), the reaction mixture was cooled down under inter atmosphere and the reaction solution was exchanged with new MBY (again 285 g) and the hydrogenation was started again.

7 cycles have been run. The following table shows the results of the cycles.

| **Cycles** | **Selectivity [%]** | **Conversion [%]** | **Yield [%]** |
|---|---|---|---|
| **1** | 96.17 | 99.78 | 96.0 |
| **2** | 96.03 | 99.99 | 96.0 |
| **3** | 95.72 | 99.98 | 95.7 |
| **4** | 95.65 | 99.99 | 95.6 |
| **5** | 96.01 | 99.99 | 96.0 |
| **6** | 96.07 | 99.98 | 96.1 |
| **7** | 95.99 | 99.99 | 96.0 |

It can be seen that the new powderous catalytic system keeps the excellent catalytic properties even after 7 cycles (without treating the catalyst after each cycle).

### Example 3: Selective hydrogenation of Dehydrolinalool (DLL).

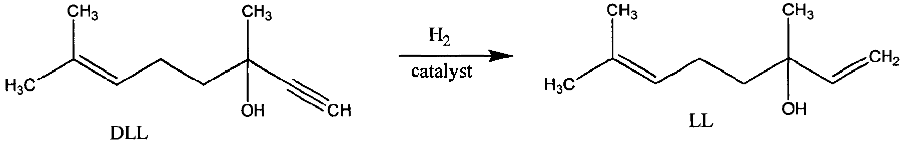

To 285 g (1.87 Mol) of DLL 1.5 g of the catalyst of Example 1 was added under stirring. The reaction was carried out at 55°C and 4 bar pressure for about 9 hours.

At the end of the reaction the selectivity of the reaction was 94.1 % and the conversion was 98.09%.

It can be seen that the new powderous catalyst has excellent properties as a catalyst for selective hydrogenations.

### Example 4: Selective hydrogenation of dehydrolinalyl acetate (DLA)

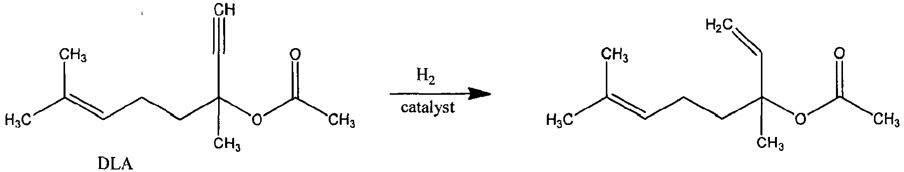

To 285g (1.5 Mol) of DLA 1.5 g of the powderous catalytic system of Example 1 was added under stirring. The reaction was carried out at 40°C and 4 bar pressure for about 34 hours.

At the end of the reaction the selectivity of the reaction was 89.53% and the conversion was 98.67%.

### Example 5: Selective hydrogenation of Dehydroisophytol (DIP)

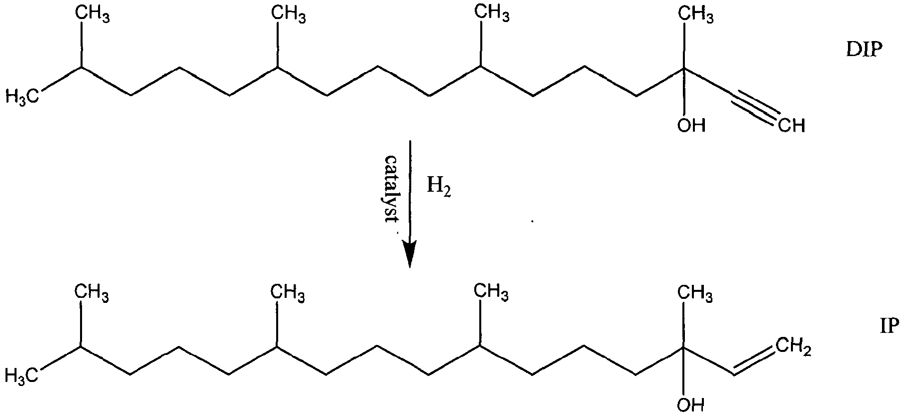

To 285 g (0.97 Mol) of DIP 1.5 g of the powderous catalytic system of Example 1 was added under stirring. The reaction was carried out at 85°C and 4 bar pressure for about 5.5 hours.

At the end of the reaction the selectivity of the reaction was 87.90 % and the conversion was 94.33%.

It can be seen that the new powderous catalytic system has excellent properties as a catalyst for selective hydrogenations.

## Claims

1. A powderous catalytic system comprising
a metal alloy carrier comprising
(i) 60 wt-% - 80 wt-%, based on the total weight of the metal alloy, of Fe, and
(ii) 12 wt-% - 25 wt-%, based on the total weight of the metal alloy, of Cr, and
(iii) 1 wt-% - 8 wt-%, based on the total weight of the metal alloy, of Ni, and
(iv) 1 wt-% - 8 wt-%, based on the total weight of the stainless steel, of Cu, and
wherein the said metal alloy is stainless steel and it is coated by a metal oxide layer, wherein the metal oxide is mixture of ZnO and Al₂O₃ in a ratio of 2:1 to 1:2, and impregnated with Pd.

2. Powderous catalytic system according to claim 1, wherein the metal alloy comprises further metals.

3. Powderous catalytic system according to any of the preceeding claims comprising between 0.1 wt-% and 50 wt-%, based on the total weight of the catalyst, of the non acidic metal oxide layer.

4. Powderous catalytic system according to any of the preceeding claims, wherein the metal oxide is mixture of ZnO and Al₂O₃ in a ratio of preferably 1:1.

5. Powderous catalytic system according to any of the preceeding claims, wherein the Pd nanoparticles have an average particle size of between 0.5 and 20 nm (measured by light scattering method).

6. Powderous catalytic system according to any of the preceeding claims, wherein the catalyst comprises between 0.001 wt-%and 5 wt-%, based on the total weight of the catalyst, of the Pd- nanoparticles.

7. Use of a powderous catalytic system according to any of claims 1 - 6 in selective catalytic hydrogenation of organic starting material.

8. Use according to claim 7, wherein the organic starting material is a compound of formula (I) 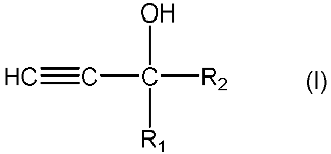 wherein
R₁ is linear or branched C₅-C₃₅ alkyl or linear or branched C₅-C₃₅ alkenyl moiety, wherein the C chain can be substituted, and
R₂ is linear or branched C₁-C₄ alkyl, wherein the C chain can be substituted.

## Patentansprüche

1. Pulverförmiges katalytisches System, umfassend einen Metalllegierungsträger, umfassend
(i) 60 Gew.-% - 80 Gew.-%, bezogen auf das Gesamtgewicht der Metallegierung, Fe und
(ii) 12 Gew.-% - 25 Gew.-%, bezogen auf das Gesamtgewicht der Metallegierung, Cr und
(iii) 1 Gew.-% - 8 Gew.-%, bezogen auf das Gesamtgewicht der Metallegierung, Ni und
(iv) 1 Gew.-% - 8 Gew.-%, bezogen auf das Gesamtgewicht des rostfreien Stahls, Cu, und
wobei die Metalllegierung rostfreier Stahl ist und mit einer Metalloxidschicht beschichtet ist, wobei es sich bei dem Metalloxid um eine Mischung von ZnO und Al₂O₃ in einem Verhältnis von 2:1 bis 1:2 handelt, und mit Pd imprägniert ist.

2. Pulverförmiges katalytisches System nach Anspruch 1, wobei die Metalllegierung weitere Metalle umfasst.

3. Pulverförmiges katalytisches System nach einem der vorhergehenden Ansprüche, umfassend zwischen 0,1 Gew.-% und 50 Gew.-%, bezogen auf das Gesamtgewicht des Katalysators, der nicht sauren Metalloxidschicht.

4. Pulverförmiges katalytisches System nach einem der vorhergehenden Ansprüche, wobei es sich bei dem Metalloxid um eine Mischung von ZnO und Al₂O₃ in einem Verhältnis von vorzugsweise 1:1 handelt.

5. Pulverförmiges katalytisches System nach einem der vorhergehenden Ansprüche, wobei die Pd-Nanopartikel eine mittlere Partikelgröße zwischen 0,5 und 20 nm (gemessen nach der Lichtstreuungsmethode) aufweisen.

6. Pulverförmiges katalytisches System nach einem der vorhergehenden Ansprüche, wobei der Katalysator zwischen 0,001 Gew.-% und 5 Gew.-%, bezogen auf das Gesamtgewicht des Katalysators, der Pd-Nanopartikel umfasst.

7. Verwendung eines pulverförmigen Katalysatorsystems nach einem der Ansprüche 1-6 bei der selektiven katalytischen Hydrierung von organischem Ausgangsstoff.

8. Verwendung nach Anspruch 7, wobei es sich bei dem organischen Ausgangsstoff um eine Verbindung der Formel (I) 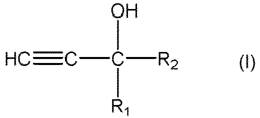 handelt, wobei
R₁ eine lineare oder verzweigte C₅-C₃₅-Alkyl- oder lineare oder verzweigte C₅-C₃₅-Alkenylgruppierung ist, wobei die C-Kette substituiert sein kann, und
R₂ lineares oder verzweigtes C₁-C₄-Alkyl ist, wobei die C-Kette substituiert sein kann.

## Revendications

1. Système catalytique pulvérulent comprenant un support d'alliage métallique comprenant
(i) 60 % en poids à 80 % en poids, sur la base du poids total de l'alliage métallique, de Fe, et
(ii) 12 % en poids à 25 % en poids, sur la base du poids total de l'alliage métallique, de Cr, et
(iii) 1 % en poids à 8 % en poids, sur la base du poids total de l'alliage métallique, de Ni, et
(iv) 1 % en poids à 8 % en poids, sur la base du poids total de l'acier inoxydable, de Cu, et
dans lequel ledit alliage métallique est de l'acier inoxydable et celui-ci est revêtu par une couche d'oxyde métallique, dans lequel l'oxyde métallique est un mélange de ZnO et de Al₂O₃ dans un rapport de 2:1 à 1:2, et imprégné avec Pd.

2. Système catalytique pulvérulent selon la revendication 1, dans lequel l'alliage métallique comprend des métaux supplémentaires.

3. Système catalytique pulvérulent selon l'une quelconque des revendications précédentes comprenant entre 0,1 % en poids et 50 % en poids, sur la base du poids total du catalyseur, de la couche d'oxyde métallique non acide.

4. Système catalytique pulvérulent selon l'une quelconque des revendications précédentes, dans lequel l'oxyde métallique est un mélange de ZnO et de Al₂O₃ dans un rapport, de préférence, de 1:1.

5. Système catalytique pulvérulent selon l'une quelconque des revendications précédentes, dans lequel les nanoparticules de Pd ont une taille de particule moyenne comprise entre 0,5 et 20 nm (mesurée par un procédé de diffusion de la lumière).

6. Système catalytique pulvérulent selon l'une quelconque des revendications précédentes, dans lequel le catalyseur comprend entre 0,001 % en poids et 5 % en poids, sur la base du poids total du catalyseur, des nanoparticules de Pd.

7. Utilisation d'un système catalytique pulvérulent selon l'une quelconque des revendications 1 à 6 dans l'hydrogénation catalytique sélective d'un matériau organique de départ.

8. Utilisation selon la revendication 7, dans laquelle le matériau organique de départ est un composé de formule (I) 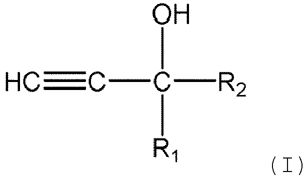 dans laquelle
R₁ est un fragment alkyle en C₅-C₃₅ linéaire ou ramifié ou alcényle en C₅-C₃₅ linéaire ou ramifié, dans lequel la chaîne de C peut être substituée, et
R₂ est alkyle en C₁-C₄ linéaire ou ramifié, dans lequel la chaîne de C peut être substituée.
